# EUROPÄISCHE PATENTSCHRIFT

(11) **EP 1 504 718 B1**
(45) Veröffentlichungstag und Bekanntmachung des Hinweises auf die Patenterteilung: **21.08.2013**
(21) Anmeldenummer: 04017054.0
(22) Anmeldetag: 20.07.2004
(51) Int. Cl.: A61B 5/15

(54) **Blutentnahmesystem**
Blood sampling system
Système de prélèvement du sang

(30) Priorität: 07.08.2003 DE 10336933
(43) Veröffentlichungstag der Anmeldung: 09.02.2005
(60) Teilanmeldung: 13000864.2
(73) Patentinhaber: Roche Diagnostics GmbH, 68305 Mannheim (DE); F. Hoffmann-La Roche AG, 4070 Basel (CH)
(72) Erfinder: Thoes, Bruno Robert, 66287 Quierschied (DE); Schabbach, Michael, 69469 Weinheim (DE); Kintzig, Hans, 67311 Tiefenthal (DE); Ruschke, Peter, Dr., 55257 Budenheim (DE); Kuhr, Dr. Hans, 68219 Mannheim (DE)
(74) Vertreter: Pfeifer, Hans-Peter

(56) Entgegenhaltungen:
- EP-A- 0 958 783
- EP-A- 1 074 219
- EP-A- 1 166 719
- EP-A- 1 384 438
- DE-A- 4 212 315
- US-A- 4 416 279
- US-A- 5 324 303

## Beschreibung

Die Erfindung betrifft ein Blutentnahmesystem zur Entnahme von Blut für Diagnosezwecke.

Um für analytisch-diagnostische Zwecke eine geringe Menge Blut aus einem Körperteil (meistens dem Finger oder dem Ohrläppchen) zu entnehmen, werden Lanzetten verwendet, die zur Erzeugung einer Wunde in das entsprechende Körperteil gestochen werden. Soweit dies manuell geschieht, ist speziell trainiertes Personal erforderlich. Dennoch ist der Einstich mit erheblichem Schmerz verbunden.

Bereits seit langem werden Blutentnahmesysteme verwendet, die aus einem Stechgerät und zugehörigen, für das jeweilige Gerät speziell angepaßten Lanzetten bestehen. In einem Gehäuse des Stechgerätes befindet sich ein Lanzettenantrieb, durch den eine Lanzette mechanisch in die Haut gestochen wird. Als Antriebselement für die Einstichbewegung dient eine Feder. Zu Beginn der Entwicklung waren sehr einfache Konstruktionen gebräuchlich, bei denen die Lanzette unmittelbar an einem Ende einer in einem länglichen Gehäuse angeordneten Druckfeder befestigt war (z.B. US Patent 4,469,110).

Derartige Blutentnahmesysteme wurden jedoch den hohen Anforderungen nicht gerecht, die zu erfüllen sind, wenn eine regelmäßige Überwachung bestimmter analytischer Werte des Blutes erforderlich ist. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Tätigkeit etc. stark schwankt) ihren Blutzuckerspiegel möglichst ständig innerhalb bestimmter Sollgrenzen zu halten. Durch wissenschaftliche Untersuchungen wurde bewiesen, daß mittels einer Intensivtherapie mit mindestens vier Blutanalysen pro Tag ein dramatischer Rückgang schwerster Spätschäden des Diabetes mellitus (beispielsweise eine Retinopathie mit resultierender Erblindung des Patienten) erreicht werden kann.

Diese Intensivtherapie setzt voraus, daß die Blutentnahme mit einem möglichst geringen Schmerz verbunden ist. Mit dem Ziel, diesbezüglich eine Verbesserung zu erreichen, wurden zahlreiche unterschiedliche Blutentnahmesysteme entwickelt.

Die Blutentnahme mittels eines Blutentnahmesystems erfordert zwei Grundfunktionen:
- Zunächst muß das Stechgerät durch Spannen der Antriebsfeder in den einstichbereiten Zustand versetzt werden. Dies wird als "Spannphase" bezeichnet.
- Danach erfolgt das Auslösen des Lanzettenantriebes, wobei die Entspannungsbewegung der Antriebsfeder den Einstich der Lanzette antreibt. Dies wird als "Vortriebsphase" bezeichnet.

Bei früheren Blutentnahmesystemen stellte die Einstichphase die kinematische Umkehr der Spannphase dar. Mit anderen Worten: Das Spannen des Antriebs erfolgte einfach dadurch, daß die Lanzette nach erfolgtem Einstich in die Ausgangslage zurückgedrückt wurde, in der die Feder gespannt war.

Bessere Ergebnisse, insbesondere hinsichtlich eines möglichst geringen Schmerzes bei gleichzeitig einfacher Bedienung, werden mit Blutentnahmesystemen erreicht, deren Lanzettenantrieb so ausgebildet ist, daß die beiden Funktionen entkoppelt sind:
- In einer Spannphase wird die Bewegung eines Betätigungselementes einer Spanneinrichtung über einen eingangsseitigen Kopplungsmechanismus derartig auf die Antriebsfeder übertragen, daß diese gespannt wird.
- In einer Vortriebsphase wird die Entspannungsbewegung der Antriebsfeder mittels eines ausgangsseitigen Kopplungsmechanismus derartig auf die Lanzette übertragen, daß diese die Einstichbewegung mit hoher Geschwindigkeit durchführt.

Die beiden Kopplungsmechanismen bestehen in der Regel aus mindestens teilweise unterschiedlichen Bauelementen. Jedenfalls sind die Bewegungsabläufe in beiden Phasen unterschiedlich (nicht nur eine kinematische Umkehr der anderen Phase).

Derartige Blutentnahmesysteme sind beispielsweise aus folgenden Publikationen bekannt:
(1) US-Patent 4,442,836
(2) US-Patent 5,318,584 und DE 42 12 315
(3) US-Patent 6,409,740
(4) US-Patent 6,419,661
(5) EP 1 254 632 A1

Trotz der umfangreichen Entwicklungsarbeiten, die zu den in diesen Dokumenten beschriebenen und zahlreichen weiteren Konstruktionen geführt haben, besteht ein großes Interesse an einem Blutentnahmesystem, das die schwierigen und teilweise gegenläufigen Anforderungen (minimales Schmerzempfinden, einfache Bedienung, kompakte, möglichst schlanke Bauweise und einfache, kostengünstige Konstruktion) gleichzeitig möglichst weitgehend erfüllt.

Zur Erfüllung dieses Erfordernisses wird ein Blutentnahmesystem gemäß Anspruch 1 vorgeschlagen.

Erfindungsgemäß ist - zusätzlich zu den Kopplungsmechanismen, die die Kraftübertragung von dem Spannelement auf die Antriebsfeder (Spannphase) bzw. von der Antriebsfeder auf die Lanzette (Vortriebsphase) bewirken - ein Bahnsteuerungsmechanismus vorgesehen. Dieser zusätzliche Bahnsteuerungsmechanismus ist mit dem Betätigungselement der Spanneinrichtung gekoppelt und steuert zumindest Teile der Bewegung des Lanzettenantriebs. Er weist zwei Teile auf, die als Glieder der Bahnsteuerung bezeichnet werden, nämlich ein Steuerbahnteil und einen Steuernocken. Die Steuerungswirkung basiert darauf, daß während der Bewegung des Lanzettenantriebs das Steuerbahnteil und der Steuernocken derartig mit dem Lanzettenantrieb zusammenwirken, daß eine Relativbewegung des Steuernockens bezüglich der Steuerbahn stattfindet, bei der der Steuernocken der Steuerbahn folgt, d.h. sie "abfährt" und dadurch eine Steuerfunktion des Lanzettenantriebs in der Spannphase und/oder in der Einstichphase bewirkt wird.

Die erforderliche Führung des Steuernockens längs der Steuerbahn kann durch unterschiedliche konstruktive Maßnahmen erreicht werden. Die Steuerbahn kann von einer Nut in dem Steuerbahnteil gebildet werden, deren Breite so auf die Abmessungen des Steuernockens abgestimmt ist, daß dieser in der Steuerbahn präzise geführt wird. Die steuernde Wirkung kann auch dadurch erreicht werden, daß die Steuerbahn als Gleitfläche ausgebildet ist, gegen die der Steuernocken unter Federwirkung gedrückt wird. In jedem Fall ist eines der Glieder mit dem Betätigungselement der Spanneinrichtung in dem Sinne gekoppelt, daß es mit diesem gemeinsam synchron bewegt wird. Die Kopplung kann starr oder beweglich sein, wobei im Falle einer beweglichen Kopplung übliche Kopplungselemente, wie beispielsweise Hebel, zur Verbindung des Betätigungselementes der Spanneinrichtung mit dem ("eingangsseitigen") Glied der Bahnsteuerung, mit dem es gekoppelt ist, verwendet werden können. Vorzugsweise ist dieses eingangsseitige Glied das Steuerbahnteil, während das ausgangsseitige Glied der Bahnsteuerung (welches mit dem Lanzettenantrieb so gekoppelt ist, daß es die steuernde Wirkung der Bahnsteuerung auf diesen überträgt) vorzugsweise von dem Steuernocken gebildet wird.

Die Relativbewegung des Steuernockens bezüglich des Steuerbahnteils kann durch unterschiedliche Kombinationen von Bewegungen der beiden Glieder der Bahnsteuerung realisiert werden. Beispielsweise kann auf einem Teilabschnitt der Relativbewegung das Steuerbahnteil bewegt werden, während der Steuernocken - bezogen auf das Gehäuse des Stechgerätes - stationär ist. In anderen Abschnitten kann das Abfahren der Steuerbahn auf einer Bewegung des Steuernockens bei stationärem Steuerbahnteil beruhen. Schließlich können auch beide Glieder der Bahnsteuerung simultan bewegt werden. Einzelheiten sind von dem jeweiligen Einzelfall abhängig. Vorzugsweise wird das Steuerbahnteil zumindest während mindestens eines Teils des gewünschten Steuerungsvorganges translatorisch bewegt, während die Bewegung des Steuernockens bevorzugt zumindest teilweise auf einer Kreisbahn verläuft.

Das Betätigungselement der Spanneinrichtung ist in der Regel als Spannknopf realisiert, der aus dem hinteren (der Einstichöffnung gegenüberliegenden) Ende eines langgestreckten Gehäuses herausragt und normalerweise durch Drücken mit dem Daumen betätigt wird. Die Erfindung ist jedoch auch zur Verwendung mit anderen Betätigungselementen der Spanneinrichtung geeignet, beispielsweise einem längs der Gehäusewand beweglichen Schieber oder einem durch Ziehen bewegbaren Betätigungselement, das beispielsweise von dem hinteren Gehäuseteil gebildet werden kann. Nachfolgend wird ohne Beschränkung der Allgemeinheit beispielhaft auf einen Spannknopf Bezug genommen.

Die Bahnsteuerung, die von den zum Spannen des Lanzettenantriebs (d.h. zur Kraftübertragung zwischen dem Spannknopf und dem Lanzettenantrieb) notwendigen mechanischen Elementen getrennt, jedoch mit dem Spannknopf des Lanzettenantriebs gekoppelt ist, ermöglicht die Realisierung vorteilhafter Funktionen:
- Dadurch, daß die Bahnsteuerung einerseits mit dem Spannknopf des Lanzettenantriebs gekoppelt, andererseits aber von der Kraftübertragung unabhängig ist, ist es möglich, dem Spannknopf (neben dem Spannen des Lanzettenantriebs) zusätzliche Funktionen, wie beispielsweise das Auslösen der Vortriebsphase (Einstichbewegung) oder das Entfernen einer gebrauchten Lanzette aus dem Stechgerät zuzuweisen. Damit wird eine "Einknopfbedienung" möglich.
- Die Bahnsteuerung kann verwendet werden, um definierte Zwischenpositionen des Spannknopfes zu realisieren, welche Zwischenstufen der Handhabung des Blutlanzettensystems kennzeichnen und dadurch dem Benutzer klare Hinweise über dessen Zustand (beispielsweise "entspannt", "gespannt") geben.
- Es ist möglich, mittels eines in der Steuerbahn vorgesehenen "Umkehrabschnittes" eine Tastschalter-artige Betätigung eines Spannknopfes zu realisieren, wie weiter unten noch näher erläutert wird.
- Mit Hilfe von ebenfalls weiter unten noch näher erläuterten Einwegabschnitten der Steuerbahn ist es möglich, sicherzustellen, daß diese nur in einer Richtung durchlaufen wird und damit eine bestimmte Folge von Schritten ohne nicht gewünschte Wiederholungen zu erreichen. Ein wichtiges Beispiel ist eine "Spannwiederholsperre", durch die ein mehrfaches Spannen des Antriebs verhindert wird.

Gemäß einer bevorzugten Ausführungsform richtet sich die Erfindung auf ein Blutentnahmesystem, das einen Lanzettenauswerfmechanismus mit einem Freischaltelement einschließt, welches so in dem Gehäuse gelagert ist, daß es zwischen einer Passivposition und einer Aktivposition verstellbar ist, wobei in der Aktivposition des Freischaltelementes der Lanzettenauswerfmechanismus aktiviert ist, so daß durch Betätigung eines Betätigungsknopfes des Lanzettenauswerfmechanismus eine benutzte Lanzette aus dem Gehäuse entfernt wird, und in der Passivposition des Freischaltelementes der Lanzettenauswerfmechanismus passiviert ist, so daß die Betätigung des Betätigungsknopfes nicht zum Auswerfen einer Lanzette führt, das in Einstichrichtung vordere Ende des Gehäuses mit der Einstichöffnung von einer Kappe gebildet wird, die zum Entfernen benutzter Lanzetten abnehmbar ist, die Kappe im aufgesetzten Zustand derartig mit dem Freischaltelement zusammenwirkt, daß es sich in der Passivposition befindet und das Freischaltelement beim Abnehmen der Kappe in die Aktivposition verstellt wird.

Der Begriff "Auswerfmechanismus" ist gebräuchlich, um eine Kombination mechanischer Elemente zu bezeichnen, durch die insgesamt die Entfernung der Lanzette aus dem Stechgerät durch Bewegen eines Betätigungsknopfes möglich wird, ohne sie berühren zu müssen. "Auswerfen" in diesem Sinne bedeutet nicht, daß die Lanzette bei der Entfernung aus dem Gerät beschleunigt wird. Vielmehr wird es in der Regel als vorteilhaft angesehen, wenn lediglich eine in dem Stechgerät vorhandene Lanzettenhalterung durch den Auswerfmechanismus geöffnet wird, so daß die Lanzette durch Schwerkraft herausfällt.

Der bevorzugte Auswerfmechanismus ist funktionsunfähig, solange die Kappe am vorderen Ende des Gehäuses des Stechgerätes aufgesetzt ist. Dadurch, daß er erst beim Abnehmen der Kappe aktiviert wird, werden mögliche Fehlbedienungen verhindert. Außerdem besteht die Möglichkeit, den Spannknopf des Lanzettenantriebs gleichzeitig als Betätigungsknopf des Auswerfmechanismus zu verwenden, ohne daß dessen übrige Funktionen, beispielsweise durch eine Einschränkung des möglichen Bewegungsweges, beeinträchtigt werden. Auch dies wird weiter unten noch näher erläutert.

Die Erfindung wird vorzugsweise bei Lanzettensystemen verwendet, deren Lanzettenantrieb einen von der Antriebsfeder angetriebenen, um eine Achse drehbaren Antriebsrotor einschließt, wobei in der Vortriebsphase des Lanzettenantriebs der Antriebsrotor, von der Antriebsfeder getrieben, eine Drehbewegung ausführt, die mittels des ausgangsseitigen Kopplungsmechanismus in die Einstichbewegung umgesetzt wird. Ein solcher Rotorantrieb wird beispielsweise bei den Lanzettensystemen verwendet, die in den oben zitierten Dokumenten (2), (3) und (4) beschrieben sind.

Besonders bevorzugt wird die vorliegende Erfindung in Verbindung mit einem speziellen Rotorantrieb eingesetzt, der in der nicht vorpublizierten EP 1 384 438 A1 (sowie den äquivalenten Patentanmeldungen US 10/445,606 und JP 149780/2003) beschrieben wird.

Bei dem dort beschriebenen Rotorantrieb stützt sich das von dem Antriebsrotor abgewandte Ende der Antriebsfeder gegen ein drehbewegliches Spannelement ab, welches zum Spannen der Antriebsfeder bei gehemmter Rotation des Antriebsrotors in die gleiche Richtung drehbar ist, in die sich der Antriebsrotor während der Vortriebsphase dreht. Das Spannelement ist während der Vortriebsphase gegen eine Rückwärtsdrehung arretiert, so daß der Antriebsrotor nach Freigabe der Hemmung eine Drehbewegung durchführt, die mittels des abtriebsseitigen Kopplungsmechanismus in die Einstichbewegung der Lanzette umgesetzt wird. Dadurch entsteht ein Antrieb, bei dem die genannten Elemente während der Spannphase und während der Vortriebsphase alternierend in die gleiche Richtung gedreht werden. Dieses Prinzip wird als "One Way Alternating Drive And Cocking, kurz OWADAC" bezeichnet.

Die Erfindung wird nachfolgend anhand von in den Figuren dargestellten Ausführungsbeispielen näher erläutert. Die dargestellten und beschriebenen Besonderheiten können einzeln oder in Kombination verwendet werden, um bevorzugte Ausgestaltungen der Erfindung zu schaffen. Es zeigen:
- Fig. 1: eine teilweise aufgeschnittene perspektivische Darstellung eines erfindungsgemäßen Blutentnahmesystems;
- Fig. 2: eine perspektivische Darstellung eines Antriebsmoduls des in dem Blutentnahmesystem gemäß Fig. 1 verwendeten Lanzettenantriebs;
- Fig. 3: eine perspektivische Explosionsdarstellung von Komponenten des Moduls von Fig. 2;
- Fig. 4: eine perspektivische Darstellung eines Moduls gemäß Fig. 2 in teilweise zusammengebautem Zustand;
- Fig. 5: eine perspektivische Darstellung eines Lanzettenhalters des Blutentnahmesystems gemäß Figur 1;
- Fig. 6: eine perspektivische Darstellung einer aus einem Antriebsmodul gemäß Fig. 2 und einem Lanzettenhalter gemäß Fig. 5 bestehenden Untereinheit des Blutlanzettensystems gemäß Figur 1;
- Fig. 7: eine perspektivische Darstellung eines Teils des in Fig. 1 dargestellten Blutentnahmesystems von der anderen Seite ("Steuerseite") her;
- Fig. 8: ein Steuerbahnteil und einen Antriebsrotor mit Steuernocken in einer perspektivisch-auseinandergezogenen Darstellung;
- Fig. 9: eine Seitenansicht auf Teile eines Auswerfmechanismus mit einem Freischaltelement in Passiv-Position;
- Fig. 10: eine Seitenansicht auf Teile eines Auswerfmechanismus mit einem Freischaltelement in Aktiv-Position

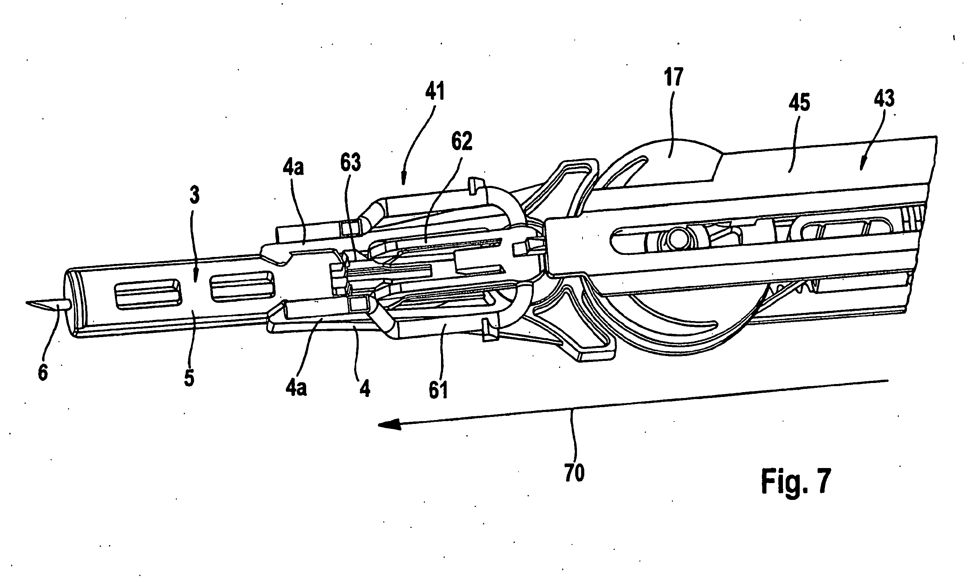

Das in den Figuren dargestellte Blutentnahmesystem 1 besteht aus einem Stechgerät 2 und Lanzetten 3. Die Halterung und Führung einer Lanzette 3 in dem Stechgerät 2 ist in Figur 7 dargestellt. Ein Lanzettenhalter 4 umgreift mit Haltezungen 4a den als Lanzettenkörper 5 bezeichneten hinteren Teil der Lanzette. Aus dem Lanzettenkörper 5 ragt eine Lanzettenspitze 6 nach vorne heraus.

Während des Einstichvorgangs wird der Lanzettenhalter 4 mittels eines als Führung dienenden (nicht dargestellten) Gehäuseteils geführt und sorgt damit indirekt für die erforderliche Führung der Lanzette auf einem vorbestimmten Einstichweg (hier längs der Hauptachse A des Stechgerätes 2). In diesem Fall wird also die Lanzette indirekt mittels des Lanzettenhalters 4 geführt.

Die Erfindung ist jedoch auch bei Stechgeräten anwendbar, bei denen die Lanzette "direkt geführt" ist, d.h. sich unmittelbar in einem die erforderliche Führung während der Einstichbewegung bildenden Teil des Gehäuses (beispielsweise einem Magazin, das eine Mehrzahl von Lanzetten enthält) befindet. Auch hierzu können nähere Informationen der EP 1 384 438 A1 entnommen werden.

In dem Gehäuse 8 des Stechgerätes 2 befindet sich ein Lanzettenantrieb 9, der dazu dient, eine Lanzette mit hoher Geschwindigkeit in Einstichrichtung (Pfeil 10) zu bewegen, bis ihre Spitze aus einer Austrittsöffnung 11 austritt, während das Stechgerät 2 mit einer die Austrittsöffnung 11 umgebenden Kontaktfläche 12 gegen ein nicht dargestelltes Körperteil gedrückt wird. Dadurch wird in dem Körperteil eine Wunde zur Entnahme von Blut erzeugt. Das Gehäuse 8 des Stechgerätes hat eine langgestreckte Form. Das Ende, an dem sich die Austrittsöffnung 11 befindet, wird hier als vorderes Ende, das gegenüberliegende Ende als hinteres Ende bezeichnet. Das vordere Ende des Gehäuses 8 wird von einer abnehmbaren Kappe 7 gebildet.

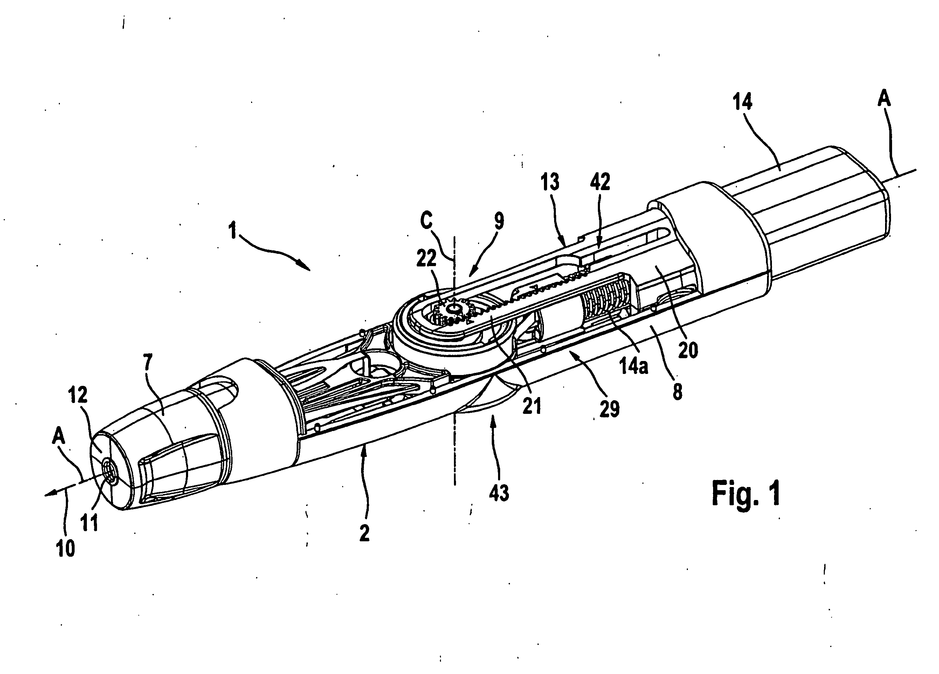

Die Figuren 1 bis 6 verdeutlichen die Konstruktionsmerkmale des Lanzettenantriebs 12, die für die Kraftübertragung während der Spannphase und während der Vortriebsphase erforderlich sind. Während der Spannphase wird die auf einen Spannknopf 14 ausgeübte Kraft mittels einer insgesamt mit 13 bezeichneten Spanneinrichtung an eine (hier in einen Antriebsmodul 15 integrierte) Antriebsfeder 16 übertragen, um diese zu spannen. Während der Vortriebsphase entspannt sich die Antriebsfeder 16 und treibt dadurch einen Antriebsrotor 17 an, dessen Drehbewegung wiederum in die translatorische Einstichbewegung des Lanzettenhalters 4 umgesetzt wird. Diese Kraftübertragung stimmt bei der vorliegenden Erfindung mit der EP 1 384 438 A1 überein.

Bestandteile des Antriebsmoduls 15 sind der Antriebsrotor 17 und ein Spannelement 19, die um eine gemeinsame Achse C drehbar sind, welche senkrecht zu der Einstichrichtung 10 und zu der Längsachse A des Stechgerätes 2 verläuft.

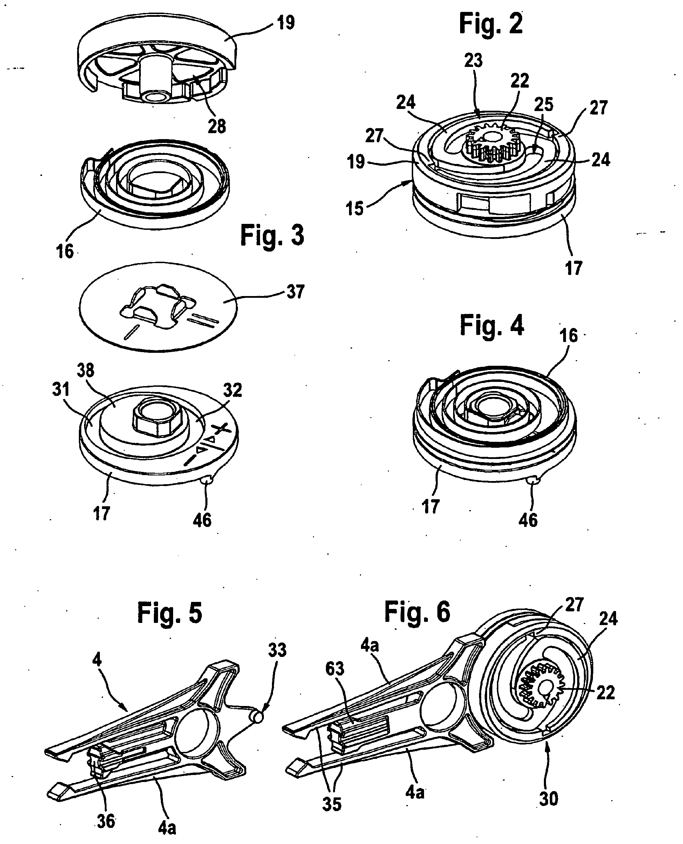

Zum Spannen des Lanzettenantriebs 9 wird der translatorisch bewegbare Spannknopf 14 und mit ihm ein Kraftübertragungsteil 20 in Einstichrichtung 10 gedrückt. Eine Zahnstange 21 ist Bestandteil des Kraftübertragungsteils 20 und treibt ein mit dem Spannelement 19 koaxiales Ritzel 22 an. Das Ritzel 22 ist mit dem Spannelement 19 über einen Freilauf 23 derart verbunden, daß beide Teile während der Spannbewegung (Bewegung des Kraftübertragungsteil 20 in Einstichrichtung) miteinander gekoppelt, hingegen bei der Rückführung des Kraftübertragungsteils 20 und des Spannknopfes 14 unter der Wirkung einer Rückstellfeder 14a entkoppelt sind. Bei der dargestellten Ausführungsform ist der Freilauf 23 mittels zweier elastischer Zungen 24 realisiert, die mit dem Ritzel 22 verbunden sind. Die Zungen 24 befinden sich in einer von dem Antriebsrotor 17 abgewandten Ausnehmung 25 des Spannelements 19 mit Anschlägen 27, an denen die Enden der Zungen 24 in der Kopplungs-Drehrichtung (in Figur 2 im Uhrzeigersinn) anliegen, während in der umgekehrten Richtung das Ritzel 22 relativ zu dem Spannelement 19 frei drehen kann.

Durch die Drehung des Spannelementes 19 wird die Antriebsfeder 16 gespannt, die als Spiralfeder ausgebildet ist und in einer dem Antriebsrotor 17 zugewandten Ausnehmung 28 des Spannelementes 19 gelagert ist. Das Kraftübertragungsteil 20 mit der Zahnstange 21, das Ritzel 22, der Freilauf 23 und das Spannelement 19 bilden insgesamt einen eingangsseitigen Kopplungsmechanismus 29, durch den in der Spannphase die auf den Spannknopf 14 ausgeübte Kraft an die Antriebsfeder 16 übertragen wird.

Der ausgangsseitige Kopplungsmechanismus 30, durch den die Kraft der Feder 16 in der Vortriebsphase an die Lanzette 3 übertragen wird, schließt eine Steuerkurve 31 ein, die von einer umlaufenden Nut 32 gebildet wird. Die Steuerkurve 31 hat in dem dargestellten Fall die Form eines zu der Achse C exzentrischen Kreises. Sie wird während der Drehung des Antriebsrotors 17 von einem Steuerzapfen 33 abgefahren, der Bestandteil des Lanzettenhalters 4 ist. Zur Fixierung einer Lanzette hat der Lanzettenhalter 4 elastische Arme 35 und ein Anschlagelement 36, deren Formgebung so auf die korrespondierende Formgebung des Lanzettenkörpers 5 abgestimmt ist, daß die Lanzetten 3 in einer exakt reproduzierbaren Longitudinalposition in dem Lanzettenhalter 4 gehalten wird.

Das hintere Ende des Lanzettenhalters 4 mit dem Steuerzapfen 33 greift in einen umlaufenden Schlitz des Antriebsmoduls 15 derartig ein, daß die Drehbewegungen der Bestandteile des Moduls 15 nicht behindert werden. Um dies sicherzustellen, ist bei der dargestellten Ausführungsform eine Trennscheibe 37 aus Metall vorgesehen, die auf einem Plateau 38 des Antriebsrotors 17 derartig aufliegt, daß zwischen ihr und den radial auswärts von der Steuerkurve 31 gelegenen Teilen des Rotors 17 ein umlaufender Spalt mit der erforderlichen Breite für den Lanzettenhalter 4 verbleibt. Bei der dargestellten Ausführungsform des Lanzettenantriebs 9 laufen dessen Bewegungsphasen gemäß dem OWADAC-Prinzip wie folgt ab:
- Während der Spannphase dreht sich das drehbewegliche Spannelement 19, angetrieben von dem Spannknopf 14 über das Kraftübertragungsteil 20 mittels der Zahnstange 21 und des Ritzels 22 in einer vorgegebenen Richtung (in den Figuren 1 und 2 im Uhrzeigersinn), wobei die Antriebsfeder 16 gespannt wird, während die Rotation des Antriebsrotors 17 (mittels des nachfolgend noch näher erläuterten Auslösemechanismus) gehemmt ist.
- In der Vortriebsphase treibt die Antriebsfeder 16 den Antriebsrotor 17 (nach Lösen der Hemmung) an, während gleichzeitig das Spannelement 19 (beispielsweise mittels einer in eine Ausnehmung des Spannelementes 19 eingreifenden nicht dargestellten elastischen Klinke) gegen eine Rückwärtsdrehung arretiert ist.

Die Figuren 7 bis 9 zeigen die wesentlichen Konstruktionsmerkmale der Bahnsteuerung 40 und des Auswerfmechanismus 41, die sich, bezogen auf den Antriebsrotor 17, auf der Seite des Stechgerätes 2 befinden, die der in Figur 1 erkennbaren Antriebsseite 42 gegenüberliegt und als Steuerseite 43 bezeichnet wird.

Die Glieder der Bahnsteuerung 40 sind ein Steuerbahnteil 45 und ein Steuernocken 46, der bei der dargestellten Ausführungsform als an dem Antriebsrotor 17 fixierter Stift ausgebildet ist. Dadurch, daß der Steuernocken an dem Antriebsrotor 17 fixiert ist, kann er sich nur auf einer Kreisbahn um dessen Achse C bewegen. Die Antriebsfeder 16 übt ein (entsprechend ihrem jeweiligen Spannungszustand unterschiedliches starkes, aber stets gleichgerichtetes) Drehmoment auf den Antriebsrotor 17 und somit auf den Steuernocken 46 (in den Figuren im Uhrzeigersinn) aus.

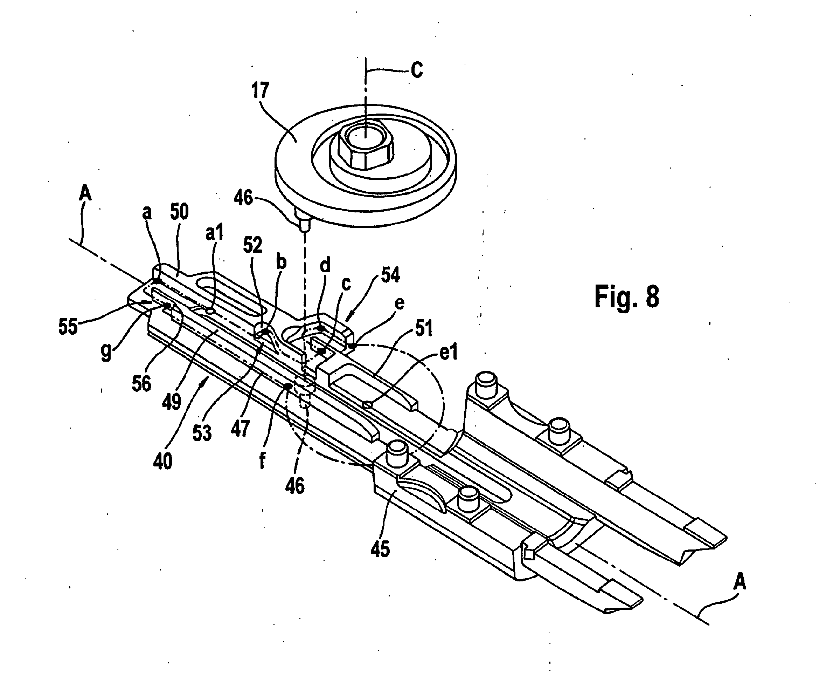

Im zusammengebauten Zustand sitzt der Antriebsrotor 17 derartig an dem Steuerbahnteil 45, daß der Steuernocken 46 sich in einer Steuerbahn 47 des Steuerbahnteils 45 befindet (in Figur 8 ist eine solche Steuerposition des Steuernockens 46 gestrichelt angedeutet). Eine Relativbewegung des Steuernockens 46 bezüglich des Steuerbahnteils 45 resultiert einerseits, wenn das Steuerbahnteil 45 parallel zu der Längsachse A des Stechgerätes 2 bewegt wird, und andererseits durch die Bewegung des Steuernockens 46 auf einer Kreisbahn um die Achse C. Die Translationsbewegung des Steuerbahnteils 45 resultiert daraus, daß es mit dem Spannknopf 14 gekoppelt ist. In der dargestellten bevorzugten Ausführungsform wird das Steuerbahnteil 45 und das Kraftübertragungsteil 20 mit der Zahnstange 21 einstückig hergestellt und etwa U-förmig gebogen, so daß in der Einbaulage die beiden Schenkel des U auf den beiden Seiten des Antriebsmoduls 15 verlaufen, nämlich das Kraftübertragungsteil 20 auf der Antriebsseite 42 und das Steuerbahnteil 45 auf der Steuerseite 43. Das hintere Ende der miteinander verbundenen Bauteile 20, 45 ist an dem Spannknopf 14 derartig befestigt, daß beide Teile bei Betätigung des Spannknopfes 14 gleichförmig translatorisch bewegt werden.

Infolge der Betätigung des Spannknopfes 14 und der Spannungswirkung der Antriebsfeder (mit resultierender Drehbewegung des Antriebsrotors 17) fährt der Steuernocken 46 die in Figur 8 strichpunktiert eingezeichnete Steuerbahn 47 ab, die durch in das Steuerbahnteil 45 eingeprägte Steuerprofile 49, 50, 51 definiert ist. Die wichtigsten Funktionspositionen der Steuerbahn sind in Figur 8 mit Buchstaben gekennzeichnet, auf die sich die nachfolgende Erläuterung bezieht:
a) Dies ist die Ausgangsposition, in der sich der Nocken 46 befindet, wenn der Lanzettenantrieb ungespannt ist und der Spannknopf 14 am weitesten aus dem Gehäuse 8 herausragt. Die Achse C des Antriebsrotors 17 befindet sich dabei in der Position a1.
b) Hier befindet sich der Steuernocken 46 in einer Tasche 52 des Steuerprofils 50, die so ausgebildet ist, daß der Steuernocken 46 nur in einer Richtung (in Fortsetzung der vorherigen Bewegung) wieder herausgefahren werden kann. Der durch die Tasche 52 definierte Abschnitt der Steuerbahn 47 wird als Einwegabschnitt 53 bezeichnet, weil der Steuernocken, wenn er in den Einwegabschnitt 53 eingefahren ist, nur in eine Richtung wieder herausgefahren werden kann, so daß eine Relativbewegung des Steuernockens 46 bezüglich des Steuerbahnteils 45 über den Einwegabschnitt 53 hinaus nur in eine Bahnrichtung der Steuerbahn 47 möglich ist. Dadurch wird eine Sicherheitsfunktion realisiert, weil ein mehrfaches Spannen des Lanzettenantriebs, das zu einer Beschädigung führen könnte, verhindert wird.
c) Beim weiteren Eindrücken des Spannknopfes 14 (und resultierender Vorwärtsbewegung des Steuerbahnteils 45) gelangt der Steuernocken 46 unter der Einwirkung der Federkraft der Antriebsfeder in diese Position vor einer von dem Steuerprofil 51 gebildeten Sperrwand, die ein weiteres Eindrücken des Spannknopfes verhindert.
d) Durch Loslassen des Spannknopfes 14 und eine resultierende Bewegung des Steuerbahnteils 45 nach hinten gelangt der Steuernocken in diese Position, die eine stabile Zwischenposition darstellt, in welcher der Lanzettenantrieb zum Auslösen, d.h. zum Start der Vortriebsphase, bereit ist. Der Spannknopf befindet sich dabei in einer Position, die sich von der Ausgangsposition (entsprechend der Steuerbahnposition a) deutlich unterscheidet und zusätzlich (beispielsweise durch eine farbige Markierung am Schaft des Spannknopfes 14) gekennzeichnet sein kann. Dadurch wird dem Benutzer eindeutig angezeigt, daß sich das Gerät im gespannten und benutzungsbereiten Zustand befindet.
   Der Abschnitt der Steuerbahn zwischen den Positionen c und e stellt einen Umkehrabschnitt 54 dar. Durch den U-förmigen Verlauf dieses Abschnitts kann der Steuernocken 46 nur nach einer zu der vorherigen Bewegung gegenläufigen Relativbewegung bezüglich des Steuerteils 45 aus dem Umkehrabschnitt 54 herausgefahren werden. Dies ermöglicht eine Tastschalter-artige Betätigung des Spannknopfes mit einer stabilen Zwischenposition.
e) Durch erneutes Niederdrücken des Spannknopfes 14 gelangt der Steuernocken 46 in diese Position, in der der Kontakt zwischen dem Steuernocken 46 und dem Steuerbahnteil 45 unterbrochen wird. Der Steuernocken ist frei, so daß der Antriebsrotor 17 eine schnelle Rotationsbewegung um die Rotorachse C ausführen kann, die sich zu diesem Zeitpunkt in der Position e1 befindet. Der Spannknopf 14 erfüllt demzufolge eine Zweifachfunktion zum Spannen und Auslösen.
f) Die Rotationsbewegung wird beendet, wenn der Steuernocken 46 in dieser Position wieder in Kontakt zu dem Steuerbahnteil 45 (hier dessen Steuerprofil 49) kommt.
g) Unter der Wirkung der Rückstellfeder 14a wird der Spannknopf 14 und mit ihm das Steuerbahnteil 45 wieder nach hinten bewegt, wobei der Steuernocken über diese Position zurück in die Ausgangsposition gelangt. Die Position g markiert wiederum einen Einwegabschnitt 55, in dem sich der Steuernocken in einer Tasche 56 befindet, durch die ein Bewegungsablauf entgegen der vorbestimmten Richtung der Steuerbahn 47 verhindert wird.

In den Figuren 8 bis 10 sind die zentralen Teile des insgesamt mit 41 bezeichneten Auswerfmechanismus zu erkennen, der bei der dargestellten Ausführungsform aus dem Spannknopf 14, dem Steuerbahnteil 45 und einem Freischaltelement 61 besteht. Diese Bauteile sind jeweils in Einstichrichtung 10 (und somit in Richtung der Achse A des Gerätes) translatorisch verschiebbar gelagert und bilden eine Kraftübertragungskette, die durch den Pfeil 70 symbolisiert wird und durch die die Bewegung eines Betätigungsknopfes des Lanzettenauswerfmechanismus auf den Lanzettenhalter 4 derartig übertragen wird, daß die Lanzette 3 freigegeben wird und durch Schwerkraft aus dem Halter 4 herausfallen kann. Bei der dargestellten Ausführungsform ist der Betätigungsknopf des Lanzettenauswerfmechanismus gleichzeitig das Betätigungselement der Spanneinrichtung, d.h. der Spannknopf 14. Dies ist zwar nicht unbedingt notwendig, aber besonders bevorzugt. Insgesamt wird damit eine Dreifachfunktion eines einzigen Betätigungsknopfes (Spannen, Auslösen, Auswerfen) möglich.

Die Freigabe der Lanzette wird bei der dargestellten Ausführungsform dadurch erreicht, daß an dem Freischaltelement 61 ausgebildete Schiebezungen 62 zwischen die elastischen Zungen 4a des Lanzettenhalters 4 und ein schräg nach außen verlaufendes Gegenlager 63 des Lanzettenhalters 4 gedrückt werden. Dadurch werden die Haltezungen 4a auseinandergedrückt und geben die Lanzette 3 frei.

Solange die abnehmbare Kappe 7, die das vordere Ende des Gehäuses 8 bildet, aufgesetzt ist, ist der Lanzettenauswerfmechanismus 41 passiviert, d.h. die Betätigung des Spannknopfes 14 führt nicht zum Auswerfen der Lanzette. Beim Abnehmen der Kappe 7 geht der Lanzettenauswerfmechanismus 41 in den aktiven Zustand über.

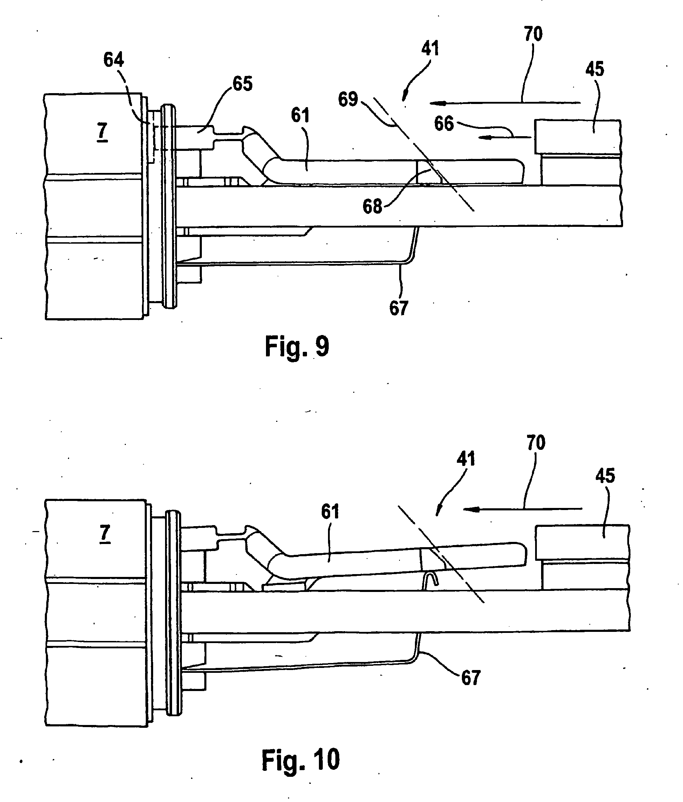

Das Umschalten zwischen aktivem und passivem Zustand wird durch eine Lageänderung des Freischaltelementes erreicht, die in den Figuren 9 und 10 dargestellt ist. In Figur 9 befindet sich das Freischaltelement in der Passivposition, in der die Kraftübertragungskette 70 zwischen dem Spannknopf und der Lanzette unterbrochen ist: Die Kappe 7 enthält ein in Figur 9 gestrichelt dargestelltes Wiederlager 64, das gegen das vordere Ende eines an dem Freischaltelement 61 ausgebildeten Betätigungsstückes 65 drückt, solange die Kappe aufgesetzt ist. Dadurch wird ein Druckstück 68 des Freischaltelementes gegen eine gerätefeste in Figur 9 nur schematisch angedeutete Schrägfläche 69 gedrückt. Das Freischaltelement wird in der in Figur 9 dargestellten Horizontalposition gehalten. Eine Verschiebung des vorderen Endes des Steuerbahnteils 45 gemäß dem Pfeil 66 wird nicht auf das Freischalt-element 61 übertragen, weil beide Teile nicht in Eingriff miteinander kommen.

Wenn hingegen die Kappe 7 abgenommen wird, entfällt der Druck des Wiederlagers 64 auf das Betätigungsstück 65 und das Freischaltelement 61 wird unter der Wirkung einer Blattfeder 67 in die in Figur 10 dargestellte Position gebracht. In dieser Position befindet sich das hintere Ende des Freischaltelementes 61 in dem Schiebeweg des Steuerbahnteils 45, so daß dessen Vorwärtsbewegung (bei Betätigung des Spannknopfes 14) auf das Freischalteelement 61 und somit auf die Lanzette übertragen wird.

## Patentansprüche

1. Blutentnahmesystem zur Entnahme von Blut für Diagnosezwecke, umfassend
ein Gehäuse (8), in dem eine Lanzette (3) entlang eines vorbestimmten Einstichwegs beweglich ist, und einen Lanzettenantrieb (9) mit einer Antriebsfeder (16) und einer Spanneinrichtung (13), wobei
- die Spanneinrichtung (13) ein Betätigungselement (14) einschließt, das über einen eingangsseitigen Kopplungsmechanismus (29) mit der Antriebsfeder (16) gekoppelt ist, wobei der Kopplungsmechanismus (29) eine Kraftübertragung von dem Betätigungselement (14) zu der Antriebsfeder (16) derartig bewirkt, daß sie in einer Spannphase der Bewegung des Lanzettenantriebs (9) bei Bewegung des Betätigungselementes (14) gespannt wird, und
- die Antriebsfeder (16) durch einen ausgangsseitigen Kopplungsmechanismus (30) mit der Lanzette (3) gekoppelt ist, wobei der ausgangsseitige Kopplungsmechanismus (30) eine Kraftübertragung von der Antriebsfeder (16) auf die Lanzette (3) derartig bewirkt, daß die Lanzette (3) in einer Vortriebsphase der Bewegung des Lanzettenantriebs (9) während der Entspannungsbewegung der Antriebsfeder (16) mit hoher Geschwindigkeit auf dem vorbestimmten Einstichweg in Einstichrichtung (10) bewegt wird, um eine Einstichbewegung auszuführen,
**dadurch gekennzeichnet, daß**
das Betätigungselement (14) der Spanneinrichtung (13) mit einer Bahnsteuerung (40) gekoppelt ist, die als Glieder der Bahnsteuerung (40) ein Steuerbahnteil (45) und einen Steuernocken (46) umfaßt, wobei die Bahnsteuerung (40) zusätzlich zu dem eingangsseitigen Kopplungsmechanismus (29), der die Kraftübertragung von dem Betätigungselement (14) zu der Antriebsfeder(16) bewirkt, und zusätzlich zu dem ausgangsseitigen Kopplungsmechanismus (30), der die Kraftübertragung von der Antriebsfeder (16) zu der Lanzette bewirkt, vorgesehen ist und der Steuernocken (46) während mindestens eines Teils der Spannphase eine Relativbewegung bezüglich des Steuerbahnteils (45) macht, bei der er die Steuerbahn (47) des Steuerbahnteils (45) abfährt und dadurch mindestens ein Teil der Bewegung des Lanzettenantriebs (9) gesteuert wird.

2. Blutentnahmesystem nach Anspruch 1, **dadurch gekennzeichnet, daß** das Steuerbahnteil (45) das dem Betätigungselement (14) der Spanneinrichtung (13) zugewandte eingangsseitige Glied der Bahnsteuerung (40) ist.

3. Blutentnahmesystem nach einem der Ansprüche 1 oder 2, **dadurch gekennzeichnet, daß** das Gehäuse (8) eine langgestreckte Form hat und das Steuerbahnteil (45) oder der Steuernocken (46) in dem Gehäuse (8) parallel zu dessen Längsachse (A) geführt und beweglich ist.

4. Blutentnahmesystem nach Anspruch 3, **dadurch gekennzeichnet, daß** das Betätigungselement (14) der Spanneinrichtung (13) ein aus dem hinteren von der Austrittsöffnung (11) der Lanzettenspitze abgewandten Ende des Gehäuses (8) herausragender Spannknopf ist.

5. Blutentnahmesystem nach einem der vorhergehenden Ansprüche, **dadurch gekennzeichnet, daß** die Steuerbahn des Steuerbahnteils (45) einen Einwegabschnitt (53) einschließt, der so ausgebildet ist, daß der Steuernocken (46), wenn er in den Einwegabschnitt (53) eingefahren ist, nur in einer Richtung aus dem Einwegabschnitt (53) herausgefahren werden kann, so daß eine Relativbewegung des Steuernockens (46) bezüglich des Steuerbahnteils (45) über den Einwegabschnitt (53) hinweg nur in eine Bahnrichtung der Steuerbahn möglich ist.

6. Blutentnahmesystem nach einem der vorhergehenden Ansprüche, **dadurch gekennzeichnet, daß** die Steuerbahn (47) des Steuerbahnteils (45) einen Umkehrabschnitt (54) einschließt der so ausgebildet ist, daß der Steuernocken (46), wenn er in den Umkehrabschnitt (54) eingefahren ist, nur nach einer zu der vorherigen Bewegung gegenläufigen Relativbewegung des Steuernockens (46) bezüglich des Steuerbahnteils (45) aus dem Umkehrabschnitt (54) herausgefahren werden kann.

7. Blutentnahmesystem nach einem der vorhergehenden Ansprüche, **dadurch gekennzeichnet, daß** die Bewegung des Lanzettenantriebs (9) während mindestens eines Teils der Spannphase bis zum Auslösen der Vortriebsphase durch die Relativbewegung des Steuernockens (46) bezüglich des Steuerbahnteils (45) gesteuert wird.

8. Blutentnahmesystem nach einem der vorhergehenden Ansprüche, **dadurch gekennzeichnet, daß** der Lanzettenantrieb einen von der Antriebsfeder (16) angetriebenen, um eine Achse (C) drehbaren Antriebsrotor (17) einschließt und in der Vortriebsphase der Bewegung des Lanzettenantriebs (9) die Drehbewegung des Antriebsrotors (17) durch den ausgangsseitigen Kopplungsmechanismus (30) in die Einstichbewegung umgesetzt wird.

9. Blutentnahmesystem nach Anspruch 8, **dadurch gekennzeichnet, daß** eines der Glieder (45,46) der Bahnsteuerung (40) derartig mit dem Antriebsrotor (17) gekoppelt ist, daß es sich synchron mit diesem dreht.

10. Blutentnahmesystem nach Anspruch 9, **dadurch gekennzeichnet, daß** das mit dem Antriebsrotor (17) gekoppelte Glied der Bahnsteuerung (40) der Steuernocken (46) ist.

11. Blutentnahmesystem nach einem der Ansprüche 8 bis 10 in Verbindung mit Anspruch 3, **dadurch gekennzeichnet, daß** das Betätigungselement (14) der Spanneinrichtung (13) mit einem in dem Gehäuse in dessen Längsrichtung verschiebbar geführten Kraftübertragungsteil (20) gekoppelt ist und die Rotationsachse (C) des Antriebsrotors quer zu der Längsachse (A) des Gehäuses verläuft.

12. Blutentnahmesystem nach Anspruch 11, **dadurch gekennzeichnet, daß** der eingangsseitige Kopplungsmechanismus (29) eine an dem Kraftübertragungsteil (20) ausgebildete Zahnstange (21) und ein mit der Zahnstange (21) kämmendes Ritzel (22) einschließt.

13. Blutentnahmesystem nach einem der Ansprüche 8 bis 12, **dadurch gekennzeichnet, daß** der ausgangsseitige Kopplungsmechanismus (30) eine Steuerkurve (31) bildende Ausnehmung (32) einschließt, in die ein Steuerzapfen (33) eingreift, wobei während der in der Vortriebsphase stattfindenden Drehbewegung des Antriebsrotors (17) der Steuerzapfen (33) die durch die Ausnehmung (32) gebildete Steuerkurve (31) abfährt und dadurch mindestens ein Teil der Bewegung der Lanzette (3) in der Vortriebsphase bestimmt wird, wobei die maximale Auslenkung der Lanzette in Einstichrichtung (10) durch einen unteren Umkehrpunkt der Steuerkurve bestimmt wird.

14. Blutentnahmesystem nach einem der Ansprüche 8 bis 13, **dadurch gekennzeichnet, daß**
sich das von dem Antriebsrotor (17) abgewandte Ende der Antriebsfeder (16) gegen ein drehbewegliches Spannelement (19) abstützt,
das Spannelement (19) zum Spannen der Antriebsfeder (16) bei gehemmter Rotation des Antriebsrotors (17) in die gleiche Drehrichtung drehbar ist, in die sich der Antriebsrotor (17) während der Vortriebsphase dreht, und
das Spannelement (19) während der Vortriebsphase gegen eine Rückwärtsdrehung arretiert ist, so daß der Antriebsrotor nach Freigabe der Hemmung eine Drehbewegung durchführt, die mittels des ausgangsseitigen Kopplungsmechanismus (30) in die Einstichbewegung der Lanzette umgesetzt wird.

15. Blutentnahmesystem nach Anspruch 14, **dadurch gekennzeichnet, daß** das drehbewegliche Spannelement (19) und der Antriebsrotor (17) um eine gemeinsame Achse (C) drehbare Bestandteile eines Antriebsmoduls (15) sind.

16. Blutentnahmesystem nach einem der Ansprüche 1 bis 15, **dadurch gekennzeichnet, daß**
es einen Lanzettenauswerfmechanismus (41) mit einem Freischaltelement (61) einschließt, welches so in dem Gehäuse (8) gelagert ist, daß es zwischen einer Passivposition und einer Aktivposition verstellbar ist, wobei in der Aktivposition des Freischaltelementes (61) der Lanzettenauswerfmechanismus (41) aktiviert ist, so daß durch Betätigung eines Betätigungsknopfes des Lanzettenauswerfmechanismus (41) eine benutzte Lanzette (3) aus dem Gehäuse (8) entfernt wird, und in der Passivposition des Freischaltelementes (61) der Lanzettenauswerfmechanismus (41) passiviert ist, so daß die Betätigung des Betätigungsknopfes nicht zum Auswerfen einer Lanzette führt,
das in Einstichrichtung vordere Ende des Gehäuses (8) mit der Einstichöffnung (11) von einer Kappe (7) gebildet wird, die zum Entfernen benutzter Lanzetten abnehmbar ist,
die Kappe (7) im aufgesetzten Zustand derartig mit dem Freischaltelement (61) zusammenwirkt, daß es sich in der Passivposition befindet und
das Freischaltelement (61) beim Abnehmen der Kappe (7) in die Aktivposition verstellt wird.

17. Blutentnahmesystem nach Anspruch 16, **dadurch gekennzeichnet, daß**
das Freischaltelement (61) ein Teil der zum Auswerfen einer Lanzette (3) notwendigen Kraftübertragungskette (70) zwischen dem Betätigungsknopf und der Lanzette ist,
das Freischaltelement (61) gleichgerichtet mit dem Betätigungsknopf verschiebbar ist, und
die Kraftübertragungskette (70) in der Passivposition des Freischaltelementes (61) unterbrochen ist.

18. Blutentnahmesystem nach einem der Ansprüche 16 oder 17, **dadurch gekennzeichnet, daß** das Betätigungselement (14) der Spanneinrichtung (13) des Lanzettenantriebs (9) zugleich der Betätigungsknopf des Lanzettenauswerfmechanismus (41) ist.

## Claims

1. Blood withdrawal system for withdrawing blood for diagnostic purposes, comprising
a housing (8) in which a lancet (3) can be moved along a pre-determined puncturing path, and
a lancet drive (9) with a drive spring (16) and a cocking mechanism (13), wherein
- the cocking mechanism (13) includes an actuation element (14), which is coupled by an input-side coupling mechanism (29) to the drive spring (16), the coupling mechanism (29) transmitting force from the actuating element (14) to the drive spring (16) such that the drive spring (16) is tensioned when the actuation element (14) is moved during a cocking phase of the motion of the lancet drive (9), and
- the drive spring (16) is coupled by an output-side coupling mechanism (30) to the lancet (3), the output-side coupling mechanism (30) transmitting force from the drive spring (16) to the lancet (3) such that the lancet (3) is driven, during a drive phase of the motion of the lancet drive (9), by the relaxing motion of the drive spring (16), at high speed along the pre-determined puncturing path in the puncturing direction (10) to perform a puncturing movement,
**characterized in that**
the actuation element (14) of the cocking mechanism (13) is coupled to a track control mechanism (40) comprising as components of the track control mechanism (40) a control track part (45) and a control traveller (46), the track control mechanism (40) being provided in addition to the input-side coupling mechanism (29), which transmits force from the actuating element (14) to the drive spring (16), and in addition to the output-side coupling mechanism (30), which transmits force from the drive spring (16) to the lancet, and the control traveller (46) performing a relative motion with respect to the control track part (45) during at least a part of the cocking phase, wherein the control traveller (46) travels along the control track (47) of the control track part (45), whereby at least a part of the motion of the lancet drive (9) is controlled.

2. Blood withdrawal system according to claim 1, **characterized in that** the control track part (45) is the input-side component of the track control mechanism (40) coupled with the actuation element (14) of the cocking mechanism (13).

3. Blood withdrawal system according to any one of claims 1 or 2, **characterized in that** the housing (8) has an elongated shape and the control track part (45) or the control traveller (46) is movably guided in the housing (8) parallel to its longitudinal axis (A).

4. Blood withdrawal system according to claim 3, **characterized in that** the actuation element (14) of the cocking mechanism (13) includes a cocking button, which projects from the rear end of the housing (8) facing away from the exit opening (11) of the lancet tip.

5. Blood withdrawal system according to any one of the preceding claims, **characterized in that** the control track of the control track part (45) includes a one-way section (53), which is designed and arranged in such a manner that the control traveller (46), once it is driven into the one-way section (53), can be driven out of the one-way section (53) only in one direction whereby a relative motion of the control traveller (46) with respect to the control track part (45) beyond the one-way section (53) is possible only in one travel direction of the control track.

6. Blood withdrawal system according to any one of the preceding claims, **characterized in that** the control track (47) of the control track part (45) includes a reversing section (54), which is designed and arranged in such a manner that the control traveller (46), once it is driven into the reversing section (54), can be driven out of the reversing section (54) only after the control traveller (46) performs a relative motion with respect to the control track part (45) in the reverse direction of its previous motion.

7. Blood withdrawal system according to any one of the preceding claims, **characterized in that** the motion of the lancet drive (9) is controlled by the relative motion of the control traveller (46) with respect to the control track part (45) during at least a part of the cocking phase until the triggering of the drive phase.

8. Blood withdrawal system according to any one of the preceding claims, **characterized in that** the lancet drive includes a drive rotor (17), which is driven by the drive spring (16) and is rotatable around an axis (C), and that during the drive phase of the motion of the lancet drive (9) the rotational motion of the drive rotor (17) is converted by the output-side coupling mechanism (30) into the puncturing motion.

9. Blood withdrawal system according to claim 8, **characterized in that** one of the components (45, 46) of the track control mechanism (40) is coupled to the drive rotor (17) in such a manner that it rotates synchronous therewith.

10. Blood withdrawal system according to claim 9, **characterized in that** the component of the track control mechanism (40) which is coupled to the drive rotor (17) is the control traveller (46).

11. Blood withdrawal system according to any one of claims 8 to 10 in combination with claim 3, **characterized in that** the actuation element (14) of the cocking mechanism (13) is coupled to a force transmission part (20), which is movably guided within the housing in its longitudinal direction, and the rotation axis (C) of the drive rotor extends transverse to the longitudinal axis (A) of the housing.

12. Blood withdrawal system according to claim 11, **characterized in that** the input-side coupling mechanism (29) includes a toothed rack (21) provided on the force transmission part (20), and a pinion gear (22) which engages the toothed rack (21).

13. Blood withdrawal system according to any one of claims 8 to 12, **characterized in that** the output-side coupling mechanism (30) includes a recess (32), which forms a guide curve (31) and is engaged by a guide pin (33), wherein, during the rotational motion of the drive rotor (17) in the drive phase, the guide pin (33) travels along the guide curve (31) formed by the recess (32), whereby at least a part of the motion of the lancet (3) in the drive phase is controlled and the maximal displacement of the lancet in the puncturing direction (10) is determined by a lower reversing point of the guide curve.

14. Blood withdrawal system according to any one of claims 8 to 13, **characterized in that**
the end of the drive spring (16) facing away from the drive rotor (17) abuts against a rotatable tensioning element (19),
the tensioning element (19) for tensioning the drive spring (16) is rotatable, while the rotation of the drive rotor (17) is inhibited, in the same rotation direction in which the drive rotor (17) rotates during the drive phase,
the tensioning element (19) is locked against a reverse motion during the drive phase such that the drive rotor performs, after release of the inhibition, a rotational motion which is transformed by the output-side coupling mechanism (30) into the puncturing motion of the lancet.

15. Blood withdrawal system according to claim 14, **characterized in that** the rotatable tensioning element (19) and the drive rotor (17) are components of a drive module (15), the components being rotatable about a common axis (C).

16. Blood withdrawal system according to any one of claims 1 to 15, **characterized in that**
the system includes a lancet ejection mechanism (41) with a release element (61) which is borne in the housing (8) in such a manner that it can be switched between a passive position and an active position, the lancet ejection mechanism (41) being activated when the release element (61) is in its active position such that actuating an actuation button of the lancet ejection mechanism (41) removes a used lancet (3) from the housing (8), and the lancet ejection mechanism (41) being inactivated when the release element (61) is in its passive position such that actuating the actuation button does not lead to the ejection of a lancet,
the front end, in the puncturing direction, of the housing (8) with the puncturing opening (11) is formed by a cap (7), which can be removed in order to remove used lancets,
the cap (7), when attached, interacts with the release element (61) such that it is in its passive position, and
the release element (61) is switched to its active position upon removal of the cap (7).

17. Blood withdrawal system according to claim 16, **characterized in that**
the release element (61) is a part of a force transmission chain (70) between the actuation button and the lancet, the force transmission chain being required for the ejection of a lancet (3),
the release element (61) can be displaced in the same direction as the actuation button, and
the force transmission chain (70) is interrupted when the release element (61) is in its passive position.

18. Blood withdrawal system according to any one of claims 16 or 17, **characterized in that** the actuation element (14) of the cocking mechanism (13) of the lancet drive (9) is also the actuation button of the lancet ejection mechanism (41).

## Revendications

1. Système de prélèvement de sang pour prélever du sang à des fins de diagnostic, comprenant
un boîtier (8), dans lequel une lancette (3) est déplaçable le long d'un trajet de piqûre prédéterminé, et
un entraînement de lancette (9) avec un ressort d'entraînement (16) et un dispositif tendeur (13),
- le dispositif tendeur (13) incluant un élément d'actionnement (14) qui est couplé au ressort d'entraînement (16) au moyen d'un mécanisme de couplage (29) côté entrée, ledit mécanisme de couplage (29) provoquant une transmission de force de l'élément d'actionnement (14) au ressort d'entraînement (16) de façon à tendre ce dernier durant une phase de tension du mouvement de l'entraînement de lancette (9) lorsque l'élément d'actionnement (14) est déplacé, et
- le ressort d'entraînement (16) étant couplé à la lancette (3) au moyen d'un mécanisme de couplage (30) côté sortie, ledit mécanisme de couplage (30) côté sortie provoquant une transmission de force du ressort d'entraînement (16) à la lancette (3) de façon à déplacer la lancette (3) durant une phase de propulsion du mouvement de l'entraînement de lancette (9) à grande vitesse sur le trajet de piqûre prédéterminé dans le sens de la piqûre (10) afin d'effectuer un mouvement de piqûre,
**caractérisé en ce que**
l'élément d'actionnement (14) du dispositif tendeur (13) est couplé à une commande de trajectoire (40) qui comprend comme organes de la commande de trajectoire (40) un élément de commande de trajectoire (45) et une came de commande (46), ladite commande de trajectoire (40) étant prévue en plus du mécanisme de couplage (29) côté entrée qui provoque la transmission de force de l'élément d'actionnement (14) au ressort d'entraînement (16), et en plus du mécanisme de couplage (30) côté sortie qui provoque la transmission de force du ressort d'entraînement (16) à la lancette (3), et ladite came de commande (46) effectuant, pendant au moins une partie de la phase de tension, un mouvement relatif par rapport à l'élément de commande de trajectoire (45), mouvement lors duquel elle suit la trajectoire de commande (47) de l'élément (45), permettant ainsi de commander au moins une partie du mouvement de l'entraînement de lancette (9).

2. Système de prélèvement de sang selon la revendication 1, **caractérisé en ce que** l'élément de commande de trajectoire (45) est l'organe côté entrée de la commande de trajectoire (40) tourné vers l'élément d'actionnement (14) du dispositif tendeur (13).

3. Système de prélèvement de sang selon l'une des revendications 1 ou 2, **caractérisé en ce que** le boîtier (8) a une forme allongée et l'élément de commande de trajectoire (45) ou la came de commande (46) est guidé(e) et se déplace dans le boîtier (8) parallèlement à l'axe longitudinal (A) de celui-ci.

4. Système de prélèvement de sang selon la revendication 3, **caractérisé en ce que** l'élément d'actionnement (14) du dispositif tendeur (13) est un bouton tendeur qui déborde de l'extrémité arrière du boîtier (8) opposée à l'orifice de sortie (11) de la pointe de lancette.

5. Système de prélèvement de sang selon l'une des revendications précédentes, **caractérisé en ce que** la trajectoire de commande de l'élément de commande de trajectoire (45) inclut un tronçon à voie unique (53) qui est conçu de telle manière que la came de commande (46), lorsqu'elle est rentrée dans le tronçon à voie unique (53), ne peut en ressortir que dans un sens, de sorte qu'un mouvement relatif de la came de commande (46) par rapport à l'élément de commande de trajectoire (45) au-delà du tronçon à voie unique (53) n'est possible que dans un sens de la trajectoire de commande.

6. Système de prélèvement de sang selon l'une des revendications précédentes, **caractérisé en ce que** la trajectoire de commande (47) de l'élément de commande de trajectoire (45) inclut un tronçon d'inversion (54) qui est conçu de telle manière que la came de commande (46), lorsqu'elle est rentrée dans le tronçon d'inversion (54), ne peut en ressortir qu'après un mouvement relatif en sens contraire du mouvement précédent de la came de commande (46) par rapport à l'élément de commande de trajectoire (45).

7. Système de prélèvement de sang selon l'une des revendications précédentes, **caractérisé en ce que** le mouvement de l'entraînement de lancette (9) est déterminé, pendant au moins une partie de la phase de tension jusqu'au déclenchement de la phase de propulsion, par le mouvement relatif de la came de commande (46) par rapport à l'élément de commande de trajectoire (45).

8. Système de prélèvement de sang selon l'une des revendications précédentes, **caractérisé en ce que** l'entraînement de lancette inclut un rotor d'entraînement (17) entraîné par le ressort d'entraînement (16) et mobile autour d'un axe (C), et durant la phase de propulsion du mouvement de l'entraînement de lancette (9), le mouvement de rotation du rotor d'entraînement (17) est transformé en mouvement de piqûre par le mécanisme de couplage (30) côté sortie.

9. Système de prélèvement de sang selon la revendication 8, **caractérisé en ce que** l'un des organes (45, 46) de la commande de trajectoire (40) est couplé au rotor d'entraînement (17) de telle sorte qu'il tourne en synchronisme avec ce dernier.

10. Système de prélèvement de sang selon la revendication 9, **caractérisé en ce que** l'organe de la commande de trajectoire (40) couplé au rotor d'entraînement (17) est la came de commande (46).

11. Système de prélèvement de sang selon l'une des revendications 8 à 10 en liaison avec la revendication 3, **caractérisé en ce que** l'élément d'actionnement (14) du dispositif tendeur (13) est couplé à un élément de transmission de force (20) guidé en translation dans le boîtier dans le sens longitudinal de celui-ci, et l'axe de rotation (C) du rotor d'entraînement s'étend transversalement à l'axe longitudinal (A) du boîtier.

12. Système de prélèvement de sang selon la revendication 11, **caractérisé en ce que** le mécanisme de couplage (29) côté entrée inclut une crémaillère (21) réalisée sur l'élément de transmission de force (20) et un pignon (22) engrenant avec la crémaillère (21).

13. Système de prélèvement de sang selon l'une des revendications 8 à 12, **caractérisé en ce que** le mécanisme de couplage (30) côté sortie inclut un évidement (32) formant une came de commande (31) et dans lequel s'engage un tenon de commande (33), ledit tenon de commande (33), pendant le mouvement de rotation du rotor d'entraînement (17) se produisant durant la phase de propulsion, suivant la came de commande (31) formée par l'évidement (32), permettant ainsi de déterminer au moins une partie du mouvement de la lancette (3) durant la phase de propulsion, l'excursion maximale de la lancette dans le sens de la piqûre (10) étant déterminée par un point d'inversion bas de la came de commande.

14. Système de prélèvement de sang selon l'une des revendications 8 à 13, **caractérisé en ce que**
l'extrémité du ressort d'entraînement (16) opposée au rotor d'entraînement (17) s'appuie contre un élément tendeur (19) mobile en rotation,
l'élément tendeur (19), de manière à tendre le ressort d'entraînement (16) lorsque la rotation du rotor d'entraînement (17) est bloquée, peut tourner dans le même sens de rotation dans lequel tourne le rotor d'entraînement (17) durant la phase de propulsion, et
l'élément tendeur (19), durant la phase de propulsion, est bloqué contre une rotation inverse de sorte que le rotor d'entraînement, après libération du blocage, effectue un mouvement de rotation qui est transformé en mouvement de piqûre au moyen du mécanisme de couplage (30) côté sortie.

15. Système de prélèvement de sang selon la revendication 14, **caractérisé en ce que** l'élément tendeur (19) mobile en rotation et le rotor d'entraînement (17) sont des composants, mobiles autour d'un axe commun (C), d'un module d'entraînement (15).

16. Système de prélèvement de sang selon l'une des revendications 1 à 15, **caractérisé en ce que**
il inclut un mécanisme d'éjection de lancette (41) doté d'un élément de libération (61) lequel est logé dans le boîtier (8) avec possibilité de réglage entre une position passive et une position active, dans la position active de l'élément de libération (61) le mécanisme d'éjection de lancette (41) étant activé, de sorte que par actionnement d'un bouton d'actionnement du mécanisme d'éjection de lancette (41) une lancette (3) utilisée est retirée du boîtier (8), et dans la position passive de l'élément de libération (61) le mécanisme d'éjection de lancette (41) est passivé, de sorte que l'actionnement du bouton d'actionnement n'entraîne pas l'éjection d'une lancette,
l'extrémité avant du boîtier (8) dans le sens de la piqûre, pourvue de l'orifice de piqûre (11), est formée par un capuchon (7), lequel est amovible pour retirer les lancettes utilisées,
le capuchon (7), lorsqu'il est posé, coopère avec l'élément de libération (61) de sorte que celui-ci se trouve en position passive, et
l'élément de libération (61) passe en position active en ôtant le capuchon (7).

17. Système de prélèvement de sang selon la revendication 16, **caractérisé en ce que**
l'élément de libération (61) fait partie d'une chaîne de transmission de force (70) nécessaire pour l'éjection d'une lancette (3), entre le bouton d'actionnement et la lancette,
l'élément de libération (61) peut coulisser dans la même direction que le bouton d'actionnement, et
la chaîne de transmission de force (70) est interrompue en position passive de l'élément de libération (61).

18. Système de prélèvement de sang selon l'une des revendications 16 ou 17, **caractérisé en ce que** l'élément d'actionnement (14) du dispositif tendeur (13) de l'entraînement de lancette (9) est en même temps le bouton d'actionnement du mécanisme d'éjection de lancette (41).
